# EUROPEAN PATENT APPLICATION

(11) **EP 1 839 690 A1**
(43) Date of publication of application: **03.10.2007**
(21) Application number: 07105050.4
(22) Date of filing: 27.03.2007
(51) Int. Cl.: A61M 1/00

(54) **Fluidic cassette detection mechanism**

(30) Priority: 31.03.2006 US 787970 P
(71) Applicant: Alcon, Inc., 6331 Hünenberg (CH)
(72) Inventor: Williams, David Lloyd, Newport Beach, CA 92663 (US); Gerrick, Edwin K., El Cajon, CA 92021 (US)
(74) Representative: Hanna, Peter William Derek

(57) **Abstract**

A fluidic cassette detection mechanism is used for detecting the presence of a cassette (12) at a certain position within a fluidic module (10). When the cassette is inserted into the fluidic module to a certain position and orientation, the detector mechanism senses the presence of the fluidic cassette and provides an appropriate signal to the control system (28) software. The system software can then command the closing of a clamping mechanism which holds the cassette during a procedure. The fluidic cassette detector mechanism can comprise a number of mechanical probes (22), optical interrupt switches (24), and miscellaneous components including springs and screws. The probes (22) extend beyond the face of the fluidic module (10) faceplate and beyond the rear stops of the clamps. When the fluidic cassette is inserted into the fluidic module (10) far enough to sufficiently move the probes, the probes trip the switches (24) to signal that the cassette is in the correct position and orientation for the clamping motion to begin. The correct position and orientation arc sensed due to the use of the probes (22) in the mechanism. The probes are spaced sufficiently apart on a diagonal, and raised beyond the rear stops of the cassette clamps, such that when both probes are moved appropriately, the cassette must be acceptably parallel to the clamps, and in the correct position, for the cassette clamping motion to begin.

## Description

### TECHNICAL FIELD OF THE INVENTION

The present invention relates generally to fluidic management systems and, more particularly, to fluidic cassettes. Even more particularly, the present invention relates to a system and method operable to detect the presence and position of a fluidics cassette.

### BACKGROUND OF THE INVENTION

Peristaltic pumps offer many advantages over other pumping systems. Primarily, peristaltic pumps offer increased cleanliness. Such pumps have no valves, seals or glands, and the fluid being pumped only contacts the interior of a flexible tube or flexible flow path. This greatly reduces the risk of contaminating fluid to be pumped or fluid contaminating the pump itself. Within a peristaltic pump fluid is drawn into a flexible tube or flexible flow path and trapped between two shoes or rollers before finally being expelled from the pump. The complete closure of the flexible tubing or flow path is squeezed between the shoes or rollers to provide a positive displacement action and prevent backflow, eliminating the need for check valves when the pump is running. Peristaltic pumps have a variety of applications including medical, pharmaceutical, chemical, or any other industry or any other like application where non-contamination of or by pumped fluid is important. However, the flexible hose or flow path within the pump can be dislodged within the pump, creating a situation where the metered action of the peristaltic pump is defeated or potentially allowing backflow. Therefore, an improved means of preventing free flow or backflow within the flexible flow path is desirable.

The advantages of peristaltic pumps are that the components of the pump may be chosen when the integrity of the media is a requirement of the application since the fluid type does not contact any internal parts. Seals and valves are not needed as in other pumps. Many peristaltic pumps come with wash down capabilities and/or IP54 or IP55 ratings.

For proper operation of a peristaltic pump and related fluidic systems, particularly in surgical equipment applications, cassettes are often used to contain the fluid pathway. Proper positioning of these cassettes is required to ensure the proper metering of fluids with these systems. Therefore, a need exists for a method and system for detecting the presence and proper positioning of a fluidics cassette within a fluidics management system.

### SUMMARY OF THE INVENTION

Embodiments to the present invention provide a fluidic cassette detection mechanism that substantially addresses the above identified need as well as other needs. The fluidic cassette detection mechanism detects the presence of a cassette at a certain position within a fluidic module. When the cassette is inserted into the fluidic module to a certain position and orientation, the detector mechanism senses the presence of the fluidic cassette and then provides an appropriate signal to control system software. The control system software can then command the closing of the mechanism, which holds the cassette during a procedure. The fluidic cassette detector mechanism comprises a number of mechanical probes, optical interrupt switches, and miscellaneous components including springs and screws. The probes extend beyond the face of the fluidic module faceplate and beyond the rear stops of a set of holding clamps. When the fluidic cassette is inserted into the fluidic module far enough to sufficiently move the probes, the probes trip the switches to signal that the cassette is in the correct position and orientation for a clamping motion to begin. The correct position and orientation are sensed due to the use of probes in the mechanism. The probes are spaced sufficiently apart on a diagonal, and raised beyond the rear stops of the cassette clamps, such that when both probes are moved appropriately, the cassette must be acceptably parallel to the clamps, and in the correct position, for the cassette clamping motion to begin.

### BRIEF DESCRIPTION OF THE DRAWINGS

For a more complete understanding of the present invention and the advantages thereof, reference is now made to the following description taken in conjunction with the accompanying drawings in which like reference numerals indicate like features and wherein:
FIG. 1 provides an isometric view of a fluidic module coupled to a fluidic cassette in accordance with an embodiment of the present invention;
FIG. 2 provides a functional diagram of a fluidic cassette displacing mechanical probes in accordance with an embodiment of the present invention;
FIG. 3 depicts how the positioning of the mechanical probes within the fluidic module requires that the fluidic cassette be properly positioned to generate a detect signal; and
FIG. 4 is a logic flow diagram associated with a method of positioning and securing a fluidic cassette to a fluidic module in accordance with one embodiment of the present invention.

### DESCRIPTION OF THE INVENTION

Preferred embodiments of the present invention are illustrated in the FIGs., like numerals being used to refer to like and corresponding parts of the various drawings.

Embodiments of the present invention provide a fluidic cassette detection mechanism that can detect the presence of a fluidic cassette at a certain position within a fluidic module. When the cassette is inserted into the fluidic module to a certain position and orientation, the detector mechanism senses the presence of the fluidic cassette and provides an appropriate signal to the system software. The system software can then command the closing of a clamping mechanism, which holds the cassette during a procedure. The fluidic cassette detector mechanism comprises a number of mechanical probes, optical interrupt switches, and miscellaneous components including springs and screws. The probes extend beyond the face of the fluidic module faceplate and beyond the rear stops of the clamping mechanism clamps. When the fluidic cassette is inserted into the fluidic module far enough to sufficiently move the probes, the probes trip the switches to signal that the cassette is in the correct position and orientation for clamping motion to begin. The correct position and orientation are sensed due to the use of probes in the mechanism. The probes are spaced sufficiently apart on a diagonal, and raised beyond the rear stops of the cassette clamps, such that when both probes are moved appropriately, the cassette must be acceptably parallel to the clamps, and in the correct position, for the cassette clamping motion to begin.

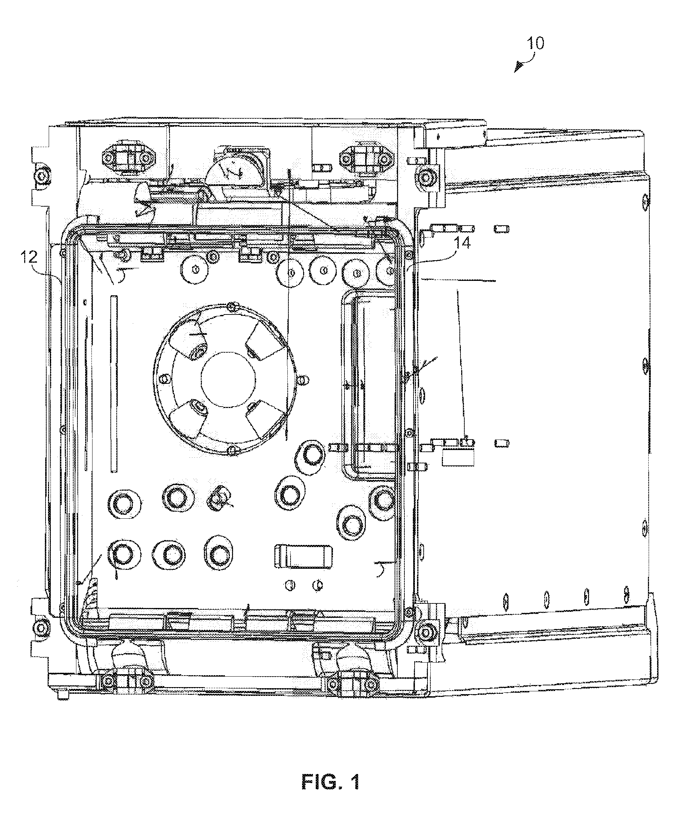

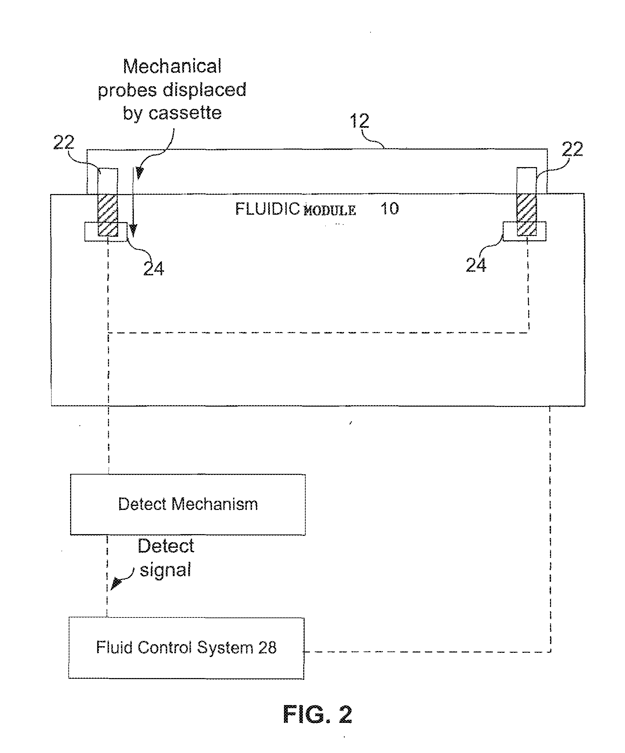

FIG. 1 provides an isometric view of a fluidic module 10 coupled to a fluidic cassette 12 in accordance with an embodiment of the present invention. Fluidic module 10 is operable to receive fluidic cassette 12. Cassette 12 may be placed on the face or surface of fluidic module 10 proximate to a cassette receptacle 14. A detector mechanism may be used to sense the presence of the fluidic cassette 12. A control module, discussed with reference to FIG. 2, may receive a cassette detection signal from the detector mechanism.

FIG. 2 provides a functional diagram of a fluidic cassette displacing mechanical probes in accordance with an embodiment of the present invention. Additionally, functional blocks are provided in FIG. 2 to further describe the function and operation of the detector mechanism. The detector mechanism (i.e., its mechanical probes) may extend beyond the face of the fluidic module 10 discussed with reference to FIG. 1. This detector mechanism may include a number of mechanical probes 22. In one embodiment, this may be two mechanical probes that are placed diagonally across the receptacle. Mechanical probes 22 are displaced when the fluidic cassette 12 is properly positioned with respect to the receptacle of the fluidic module 10.

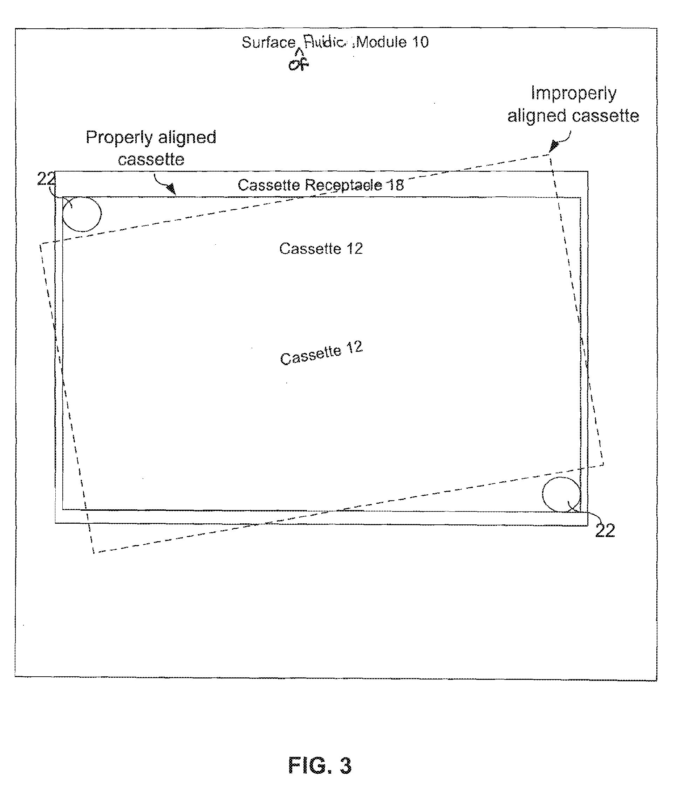

FIG. 3 illustrates how the positioning of the mechanical probes within the fluidic module requires that the fluidic cassette 12 be properly positioned to generate a detection signal. By placing the two mechanical probes 22 diagonally across, in this case, a rectangular cassette receptacle, displacement of the diagonal positioning of the mechanical probes 22 requires a proper alignment and positioning of the fluidic cassette 12. Both proper and improper alignment of the cassette 12 are shown in FIG. 3. The improper alignment would not displace both mechanical probes 22. Further, the arrangement of mechanical probes 22 is operable to ensure the fluidic cassette 12 is fully pushed into receptacle 18, as both probes 22 will not fully displace if only one side of the fluidic cassette 12 is fully pushed into receptacle 18.

The mechanical probes 22, when displaced, may generate a detect signal. This may be done by having the mechanical probes 22 interrupt an optical signal, as detected by an optical interrupt switch 24. Other switching mechanisms known to those having skill in the art may be used as well. The detect signal received by the fluidic module control system 28 may be used to initiate a closing command or clamping action that secures the fluidic cassette 12 to fluidic module 10. Additionally, the fluidic module control system 28 may initiate other functions within the fluidic cassette 12 and fluidic module 10.

Another embodiment of the present invention comprises a fluidic module such as that of the ALCON INFINITI^{™} Vision System. Such a system provides a Fluidics Management System (FMS) for ophthalmic surgery that may be used to accurately meter fluids. When the fluidic module 10, such as that shown in FIG. 1, receives a fluidic cassette 12 within a receptacle on the face or surface of the fluidic module 10, mechanical probes distributed across the receptacle generate a detect signal when displaced. A fluidic control system 28 receives the detect signal and initiates a closing command, or other functions, upon receipt of the detect signal. One reasons for requiring the accurate positioning of the fluidic cassette is to ensure the proper metering of fluids,

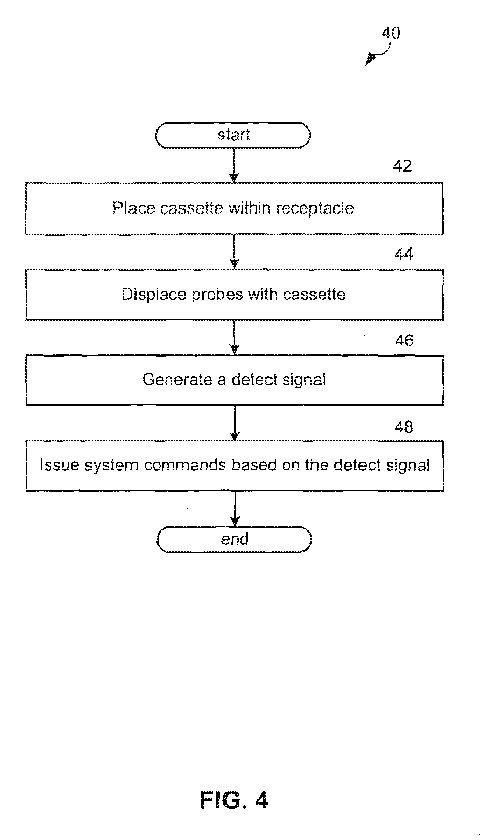

The fluidic control system may be a single processing device or a plurality of processing devices. Such a processing device may be a microprocessor, micro-controller, digital signal processor, microcomputer, central processing unit, field programmable gate array, programmable logic device, state machine, logic circuitry, analog circuitry, digital circuitry, and/or any device that manipulates signals (analog and/or digital) based on operational instructions. The memory within the fluidic control system may be a single memory device or a plurality of memory devices. Such a memory device may be a read-only memory, random access memory, volatile memory, non-volatile memory, static memory, dynamic memory, flash memory, cache memory, and/or any device that stores digital information. Note that when the fluidic control system implements one or more of its functions via a state machine, analog circuitry, digital circuitry, and/or logic circuitry, the memory storing the corresponding operational instructions may be embedded within, or external to, the circuitry comprising the state machine, analog circuitry, digital circuitry, and/or logic circuitry. The memory stores, and the processing modules executes, operational instructions corresponding to at least some of the steps and/or functions illustrated in FIG. 4.

FIG. 4 is a logic flow diagram associated with a method of positioning and securing a fluidic cassette 12 to a fluidic module 10 in accordance with an embodiment of the present invention. Processes 40 begin with the placing of a fluidic cassette 12, at step 42, within a receptacle 18 of the fluidic module 10. As the fluidic cassette 12 is properly placed within the receptacle 10, a number of mechanical probes 22 distributed across the receptacle 10 may be displaced by the fluidic cassette 12 at step 44. The displacement of these mechanical probes 22 may generate a detect signal at step 46. The detect signal may be used by a control system 28 in order to initiate a closing command at step 48, wherein the closing command initiates a clamping action that may secure the fluidic cassette 12 to the fluidic module 10.

As one of average skill in the art will appreciate, the term "substantially" or "approximately", as may be used herein, provides an industry-accepted tolerance to its corresponding term. Such an industry-accepted tolerance ranges from less than one percent to twenty percent and corresponds to, but is not limited to, component values, integrated circuit process variations, temperature variations, rise and fall times, and/or thermal noise. As one of average skill in the art will further appreciate, the term "operably coupled", as may be used herein, includes direct coupling and indirect coupling via another component, element, circuit, or module where, for indirect coupling, the intervening component, element, circuit, or module does not modify the information of a signal but may adjust its current level, voltage level, and/or power level. As one of average skill in the art will also appreciate, inferred coupling (i.e., where one element is coupled to another element by inference) includes direct and indirect coupling between two elements in the same manner as "operably coupled". As one of average skill in the art will further appreciate, the term "compares favorably", as may be used herein, indicates that a comparison between two or more elements, items, signals, etc., provides a desired relationship. For example, when the desired relationship is that signal 1 has a greater magnitude than signal 2, a favorable comparison may be achieved when the magnitude of signal 1 is greater than that of signal 2 or when the magnitude of signal 2 is less than that of signal 1.

Although the present invention is described in detail, it should be understood that various changes, substitutions and alterations can be made hereto without departing from the spirit and scope of the invention as described.

## Claims

1. A fluidic cassette position detection mechanism, comprising:
a fluidic module (10) comprising a receptacle (18) adapted to receive a fluidic cassette (12);
a detector mechanism to sense the presence of a fluidic cassette within the receptacle, comprising a plurality of mechanical probes (22) distributed across the receptacle, such that displacement of the plurality of mechanical probes by a cassette causes a detection signal to be generated;
a fluidic module control system (28) adapted to receive the detection signal from the detector mechanism and initiate a closing command upon receipt of the detection signal.

2. The fluidic cassette detection mechanism of claim 1, wherein only full displacement of the plurality of mechanical probes (22) causes the detection signal to be generated.

3. The fluidic cassette detection mechanism of claim 1 or claim 2, wherein only the displacement of all of the mechanical probes (22) causes the detection signal to be generated.

4. The fluidic cassette detection mechanism of any of claims 1 to 3, wherein the plurality of mechanical probes (22) is diagonally distributed across the receptacle (18) of the fluidic module (10).

5. The fluidic cassette detection mechanism of any of claims 1 to 4, wherein the detector mechanism further comprises at least one optical interrupt switch (24).

6. The fluidic cassette detection mechanism of claim 5, wherein each of the mechanical probes (22) is adapted to trip an optical interrupt switch (24) in order to generate the detection signal.

7. The fluidic cassette detection mechanism of any of claims 1 to 6, wherein displacement of the mechanical probes (22) to cause the detection signal is adapted to require that a fluidic cassette (12) be properly positioned and aligned with respect to the fluidic module (10).

8. The fluidic cassette detection mechanism of any of claims 1 to 7, wherein the mechanical probes (22) extend beyond a faceplate of the fluidic module (10).

9. A fluidic module (10) in combination with a fluidic cassette (12) operable to accurately meter a fluid, comprising;
a receptacle (18) within the fluidic module (10) adapted to receive the fluidic cassette;
a fluidic cassette (12) adapted to be inserted in the receptacle and to be operably coupled to the fluidic module;
a plurality of mechanical probes (22) distributed across the receptacle, wherein the probes are adapted to cause a detect signal to be generated when the probes are displaced by the insertion of the fluidic cassette; and
a fluidic module control system (28) adapted to receive the detect signal and initiate a closing command upon receipt of the detect signal.

10. The combination of claim 9, wherein the closing command initiates the motion of a clamping mechanism adapted to secure the fluidic cassette (12) to the fluidic module (10).

11. The combination of claim 9 or claim 10, wherein the detect signal is only generated when a correct position and alignment of the inserted fluidic cassette (12) with respect to the fluidic module (10) is indicated by a sufficient or appropriate displacement of the mechanical probes (22).

12. The combination of any of claims 9 to 11, further comprising a peristaltic pump comprising a fluid pathway contained by the cassette (12).

13. A method to accurately position and secure a fluidic cassette (12) to a fluidic module (10), comprising:
placing (42) a fluidic cassette within a receptacle (18) of the fluidic module adapted to receive a fluidic cassette;
displacing (44) a plurality of mechanical probes (22) distributed across the receptacle with the fluidic cassette;
generating (46) a detect signal with the plurality of mechanical probes when the plurality of mechanical probes is displaced by the fluidic cassette; and
initiating (48) a closing command to secure the fluidic cassette to the fluidic module upon receipt of the detect signal.

14. The method of claim 13, wherein only full displacement (44) of the plurality of mechanical probes (22) causes the detect signal to be generated (46).

15. The method of claim 13 or claim 14, wherein only the displacement (44) of all of the mechanical probes (22) causes the detect signal to be generated (46).

16. The method of any of claims 13 to 15, wherein the plurality of mechanical probes (22) is diagonally distributed across the receptacle (18) of the fluidic module (10).

17. The method of claim 16, wherein each of the mechanical probes (22) is adapted to trip an optical interrupt switch (24) in order to generate (46) the detection signal.

18. The method of any of claims 13 to 17, wherein displacement (44) of the mechanical probes (22) to cause the detect signal to be generated (46) requires that a fluidic cassette (12) be properly positioned and aligned with respect to the fluidic module (10).

19. The method of any of claims 13 to 18, wherein initiating (48) a closing command initiates the motion of a clamping mechanism adapted to secure the fluidic cassette (12) to the fluidic module (10).
